(19) Europäisches Patentamt
European Patent Office
Office européen des brevets

(11) Numéro de publication : **0 468 885 A1**

(12) # DEMANDE DE BREVET EUROPEEN

(21) Numéro de dépôt : **91402056.5**

(22) Date de dépôt : **24.07.91**

(51) Int. Cl.[5] : **C07D 231/16,** C07D 231/14, C07D 207/32, C07D 403/06, A61K 31/41, A61K 31/40

(30) Priorité : **26.07.90 FR 9009562**

(43) Date de publication de la demande : **29.01.92 Bulletin 92/05**

(84) Etats contractants désignés : **AT BE CH DE DK FR GB GR IT LI LU NL SE**

(71) Demandeur : **LABORATORIOS DEL DR. ESTEVE, S.A.**
**Av. Mare de Deu de Montserrat, 221**
**E-08026 Barcelona (ES)**

(72) Inventeur : **Cuberes-Altisent, Maria Rosa**
**Rue Barcelona No.2-D**
**E-08190 Sant Cugat del Valles, Barcelone (ES)**
Inventeur : **Frigola-Constansa, Jordi**
**Av. Diagonal, 299 at. la.**
**E-08013 Barcelone (ES)**
Inventeur : **Pares-Corominas, Juan**
**Padilla, 349, 30 3a**
**E-08025 Barcelone (ES)**

(74) Mandataire : **Ahner, Francis et al**
**CABINET REGIMBEAU, 26, avenue Kléber**
**F-75116 Paris (FR)**

(54) **Nouveaux dérivés de 1-diphénylméthylpipérazine, leur préparation, et leur application en tant que médicaments.**

(57) La présente invention concerne de nouveaux dérivés de 1-diphénylméthyl pipérazinyl, caractérisés en ce qu'ils répondent à la formule générale I, et leurs sels thérapeutiquement acceptables,

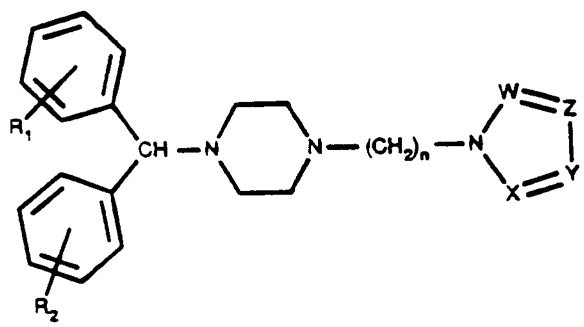

I

dans laquelle

— $R_1$ et $R_2$, égaux ou différents, représentent un atome d'hydrogène, un halogène, un radical alkyl inférieur, un radical hydroxy, un radical alkoxy, un radical carboxylate d'alkyle, un radical aryl ou aryl substitué,

— n peut avoir les valeurs 2 à 4,

— X, Y, Z et W, égaux ou différents, représentent un atome d'azote ou un atome de carbone lié à un atome d'hydrogène un halogène ou à un autre radical alkyl, aryl, carboxyalkyl, carboxylique, hydroxyle, alkyhydroxy, sulfonique et alkylsulfonique.

La présente invention concerne également le procédé de préparation des composés, et leur utilisation pour la fabrication de médicaments destinés à la prophilaxie et au traitement de diverses maladies allergiques provoquées par l'histamine.

EP 0 468 885 A1

La présente invention se rapporte à de nouveaux dérivés de 1-diphénylméthyl pipérazine, leur procédé de préparation ainsi qu'à leur application en tant que médicaments.

Les composés objet de la présente invention répondent à la formule générale I

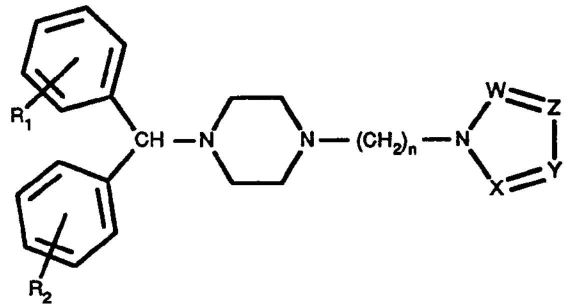

I

dans laquelle:

– $R_1$ et $R_2$, égaux ou differents, représentent un atome d'hydrogène, un halogène, un radical alkyl inférieur, un radical hydroxy, un radical alkoxy, un radical carboxylate d'alkyle, un radical aryl ou aryl substitué,

– n peut avoir les valeurs 2 à 4,

– X, Y, Z et W, égaux ou différents, représentent un atome d'azote ou un atome de carbone lié à un atome d'hydrogène, un halogène ou à un autre radical alkyl, aryl, carboxyalkyl, carboxylique, hydroxyle, alkylhydroxy, sulfonique et alkylsulfonique.

Ces nouveaux dérives, et leur sels pharmaceutiquement acceptables, présentent une très bonne activité antihistaminique.

Dans la littérature scientifique on connait déjà, depuis longtemps, des dérivés de diphénylméthyl pipérazine avec activité antihistaminique, comme chlorciclicine (Baltzly et al., J. Org. Chem., 14, 775, 1949); meclicine (Bull. Soc. Chimie Belge., 60, 282, 1951); cetyrizine (EP 58146). Mais, en général, les antihistaminiques usuels présentent des effets secondaires de stimulation ou de dépression du système nerveux central. Par contre, nous avons découvert que les dérivés de formule générale I, et leur sels pharmaceutiquement acceptables, ne présentent pas d'effets secondaires sur le système nerveux central.

Les nouveaux dérivés de formule générale I peuvent être préparés, conformément à l'invention, selon l'une quelconque des méthodes suivantes:

Méthode A .- Par réaction d'un composé de formule générale IIa

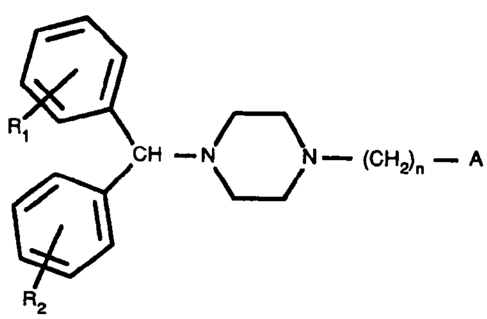

IIa

ou bien IIb

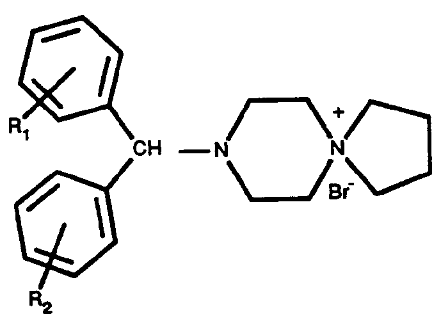

IIb

dans lesquelles $R_1$, $R_2$, et n ont les significations mentionnées précédemment, et A représente un atome d'halogène, ou un bon "groupe partant" choisi parmi le tosyloxy ou le mésyloxy, avec un composé de formule générale III

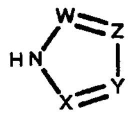

III

dans laquelle X, Y, Z et W ont les significations mentionnées précédemment.

La réaction s'effectue en présence d'un solvant adéquat, par exemple le diméthylsulfoxyde, la diméthylformamide, des alcools, des hydrocarbures, aromatiques ou non, des éthers, tels le dioxanne ou l'éther diphénylique, ou des mélanges de ces solvants. Cette réaction est avantageusement conduite en présence d'une base telle les hydroxydes, les carbonates ou les bicarbonates des métaux alcalins, ou bien d'un mélange de ces bases. On peut employer aussi les hydrures des métaux alcalins. Les températures les plus adéquates oscillent entre la température ambiante et la température de reflux du solvant, et le temps réactionnel est compris entre 1 heure et 24 heures.

Méthode B.- Par réaction d'un composé de formule générale IIa, dans laquelle A représente un radical -$NH_2$, avec le 2,5-diméthoxytétrahydrofurane.

La réaction s'effectue en présence d'un solvant adéquat, par exemple de l'acide acétique, de l'eau, des alcools, des cétones ou des mélanges de ces solvants. Les températures les plus adéquates oscillent entre la température ambiante et la température de reflux du solvant, et le temps réactionnel est compris entre quelques minutes et 24 heures.

Méthode C.- Par réaction d'un composé de formule générale IV

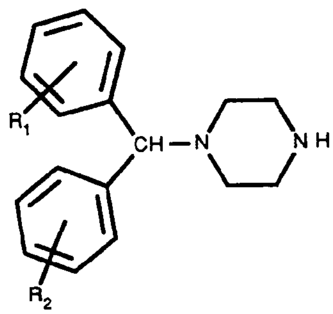

IV

dans laquelle $R_1$, $R_2$ ont les significations mentionnées précédemment, avec un composé de formule générale V

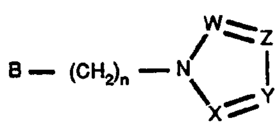

V

où X, Y, Z, W et n ont les significations mentionnées précédemment, et B représente un atome d'halogène, ou un bon "groupe partant" choisi parmi le tosyloxy ou le mésyloxy.

La réaction s'effectue on présence d'un solvant adéquat, par exemple le diméthylsulfoxyde, le diméthylformamide, des alcools, des hydrocarbures, aromatiques ou non, des éthers, tels le dioxanne ou l'éther diphénylique, ou des mélanges de ces solvants. Cette réaction est avantageusement conduite en présence d'une base telle les hydroxydes, les carbonates ou les bicarbonates des métaux alcalins, ou bien d'un mélange de ces bases. Les températures les plus adéquates oscillent entre la température ambiante et la température de reflux du solvant, et le temps réactionnel est compris entre 1 heure et 24 heures.

Dans les exemples suivants on indique la préparation des nouveaux dérivés selon l'invention. Les exemples ci-prés, donnés à simple titre d'illustration, ne doivent cependant, en aucune façon, limiter l'étendue de l'invention.

Méthode A

Exemple 1.- Préparation de 4-Bromo-1-[4-(4-diphénylméthyl pipérazinyl)butyl]pyrazole.

a) Bromure de 8-aza-5-azoniaspiro [4,5] décane-8-diphénylméthyl.

On chauffe à reflux pendant 16 heures un mélange de 10 g (39,7 mmoles) de 1-diphénylméthyl pipérazine, 11,9 g (55,1 mmoles) de 1,4-dibromobutane et 5,5 g (39,7 mmoles) de carbonate de potassium dans 60 ml de chloroforme. On réfroidit, on filtre et on évapore. On triture le résidu dans l'éther éthylique et on obtient 15,1 g de solide, avec un point de fusion 256-262°C.

$^1$H-RMN ($CDCl_3$): δ 2,2 (m,4H); 2,7 (m,4H); 3,75 (m,8H); 4,35 (s,1H); 7,2 (m,10H).

b) 4-Bromo-1-[4-(4-diphénylméthyl-1-pipérazinyl)butyl]pyrazole.

On chauffe à reflux, pendant 19 heures, un mélange de 5 g (12,9 mmoles) de bromure de 8-aza-5-azoniaspiro [4,5] décane-8-diphénylméthyl, 2,2 g (14,9 mmoles) de 4-bromo-1--H-pyrazole, et 2,74 g (19,9 mmoles) de carbonate de potassium dans 50 ml de diméthylformamide. On refroidit, on filtre et on évapore le filtrat à sec. On réprend le résidu avec du chloroforme et on lave avec de l'eau. On sèche la phase organique avec $Na_2SO_4$, on filtre et on évapore. On obtient le produit sous forme d'huile brute, on prépare son chlorhydrate avec du méthanol chlorhidrique et on crisallise dans l'éthanol-éther éthylique. On obtient 5,5 g du chlorhydrate corresponant, avec un point de fusion 184-189°C.

Les donnés spectroscopiques pour son identification sont exposées dans les Tableaux 1 et 2.

Exemple 2.- Préparation de 4-Chloro-1-[4-(4-diphénylméthyl-1-pipérazinyl)butyl]pyrazole.

La préparation est effectuée avec la même méthode que celle exposée à l'exemple 1a et 1b.

La sel avec l'acide chlorhydrique est préparée dans l'éthanol-éther éthylique, avec un point de fusion 173-176°C.

Les données spectroscopiques pour son identification sont exposées dans les tableaux 1 et 2.

Exemple 3.- Préparation de 4-Bromo-1-[3-(4-diphénylméthyl-1-pipérazinyl)propyl]pyrazole.

a) 1-(3-chloropropyl)-4-diphénylméthyl pipérazine.

On met à reflux pendant 16 heures un mélange de 5 g (19,8 mmoles) de 1-diphénylméthyl pipérazine, 3,75 g (23,8 mmoles) de 1-bromo-3-chloropropane et 3,3 g (24 mmoles) de carbonate de potassium dans 100 ml de chloroforme. On réfroidit, on filtre et on évapore le filtrat à sec. On purifie le résidu brut obtenu sur une colonne chromatographique de sillice (éluant: acétate d'éthyle) et on obtient ainsi 1,25 g de 1-(3-chloropropyl)-4-diphénylméthyl pipérazine.

$^1$H-RMN ($CDCl_3$): δ 1,9 (m,2H); 2,35 (m,10H); 3,45 (t,2H); 4,15 (s,1H); 7,2 (m,10H).

b) 4-Bromo-1-[3-(4-diphénylméthyl-1-pipérazinyl)propyl] pyrazole.

La préparation est effectuée avec une procédure pareille à celle exposée à l'exemple 1b.

Le produit est purifié sur une colonne chromatographique de sillice (éluant: chloroforme-méthanol 95:5). On prépare son chlorhydrate en éthanol - chlorhydrique , que presente un point de fusion de 106-110°C.

Les données spectroscopiques pour son identification sont exposées dans les Tableaux 1 et 2.

Exemple 4.- Préparation de 1-[4-(4-Diphénylméthyl-1-pipérazinyl)butyl]-4-carboxy pyrazole.

La préparation s'effectue avec une procédure pareille à l'exposée dans les exemples 1a et 1b, et on obtient le 1-[4-(4-Diphénylméthyl-1-pipérazinyl)butyl]-4-éthyloxycarbonylpyrazole, avec un point de fusion de 70-75°C. $^1$H-RMN ($CDCl_3$): δ 1,31 (t,3H); 1,48 (m,2H); 1,86 (m,2H); 2,41 (m,10H); 4,08 (t,2H); 4,20 (s,1H); 4,30 (t,2H); 7,12-7,46 (m,10H); 7,87 (d,2H). IR (KBr): 1708, 1552, 1237, 1152, 773, 706 $cm^{-1}$

L'ester précédemment préparé est hydrolysé par traitement d'une solution dans l'ethanol, pendant 15 heures à température ambiante, avec de la soude à 10%. On évapore l'alcool et la solution aqueuse est neutralisée avec de l'acide chlorhydrique. On extrait avec du chloroforme, on sèche avec $Na_2SO_4$, on filtre et on évapore. On obtient ainsi l'acide correspondant avec un point de fusion de 102-105°C.

Son chlorhydrate cristallise dans l'éthanol-éther éthylique, avec un point de fusion de 148-152°C.

Les données spectroscopiques pour son identification sont exposées dans les Tableaux 1 et 2.

Exemple 5.- Préparation de 1-[4-(4-Diphénylméthyl-1--pipérazinyl)butyl]-4-méthyl pyrazole.

La préparation est effectuée avec une procédure pareille à celle exposée dans les exemples 1a et 1b.

Le sel avec l'acide maléique est préparé dans l'éthanol-éther éthylique, avec un point de fusion 122-126°C.
Les données spectroscopiques pour son identification sont exposées dans les Tableaux 1 et 2.

Exemple 6.- Préparation de 1-[4-(4-Diphénylméthyl-1-pipérazinyl)butyl]imidazole.

La préparation est effectuée avec une procédure pareille à celle exposée aux exemples 1a et 1b. Le produit est purifié sur une colonne chromatographique de sillice (éluant: chloroforme-méthanol 95:5) en forme d'huile.
On prépare le sel avec l'acide maléique dans l'éthanol-acétate d'éthyle, avec un point de fusion 146-149°C.
Les données spectroscopiques pour son identification sont exposées dans les Tableaux 1 et 2.
Exemple 7.- Préparation de 1-[4-(4-Diphénylméthyl-1--pipérazinyl)butyl]-1,2,4-triazole.
La préparation est effectuée avec une procédure pareille à celle exposée aux exemples 1a et 1b, et on purifie sur une colonne chromatographique de sillice (éluant: chloroforme-méthanol 93:7). On obtient le dérivé sous forme d'huile. On prepare son chlohydrate et on cristallise dans l'éthanol-acétate d'éthyle, avec un point de fusion de 201-203°C.
Les données spectroscopiques pour son identification sont exposées dans les Tableaux 1 et 2.

Méthode B

Exemple 8.- Préparation de 1-[4-(4-Diphénylméthyl-1--pipérazinyl)butyl]pyrrole.

On chauffe à reflux, pendant 20 minutes une solution de 1,28 g (3,96 mmoles) de 1-(4-aminobutyl)-4-diphénylméhtyl pipérazine et 0,77 g (5,8 mmoles) de 2,5-diméthoxytétrahydrofurane dans 25 ml d'acide acétique. On refroidit, on verse sur de l'eau glacée, on neutralise avec $NaHCO_3$ et on extrait avec du chloroforme. On sèche avec $Na_2SO_4$, et on évapore sous vide à sec. On obtient 1,7 g d'huile brute qui est purifiée sur une colonne chromatographique de sillice (éluant: acétate d'éthyle). On obtient 0,95 g de 1-[4-(4--Diphénylméthyl-1-pipérazinyl)butyl]pyrrole en forme solide, avec point de fusion 80-84°C.
On prépare le sel avec l'acide maléique dans l'éthanol-éther éthylique, avec un point de fusion 136-140°C.
Les donnés spectroscopiques pour son identification sont exposées dans les Tableaux 1 et 2.

Méthode C

Exemple 4.- Préparation de 1-[4-(4-Diphénylméthyl-1-pipérazinyl)butyl]-4-carboxy pyrazole.

On met à reflux pendant 4 heures un mélange de 6,3 g (25 mmoles) de diphénylméthyl pipérazine, 6,87 g (25 mmoles) de 1-(4-bromobutyl)-4-éthyloxycarbonylpyrazole, 5,17 (37,5 mmoles) de carbonate de potassium et 5,06 g (33,7 mmoles) de iodure de sodium dans 100 ml de méthyl éthyl cétone. On refroidit, on filtre et on évapore le filtrat à sec. On réprend le résidu avec du chloroforme et de l'eau, on sèche la phase organique avec $Na_2SO_4$, on filtre et on évapore sous vide.Le produit résultant est purifié sur une colonne chromatographique de sillice (éluant: chloroforme-méthanol 95:5) et on obtient ainsi 7,4 g de 1--[4-(4-Diphénylméthyl-1-pipérazinyl)butyl]-4-éthyloxycarbonylpyrazole, avec point de fusion 70-74°C.
Les données spectroscopiques du composé sont les mêmes déjà exposées dans l'exemple 4 de la méthode A.
Cet ester est hydrolysé avec une procédure semblable à celle exposée dans l'exemple 4 de la méthode A et on obtient l'acide avec les mêmes données spectroscopiques exposées dans les Tableaux 1 et 2.

Exemple 8.- Préparation de 1-[4-(4-Diphénylméthyl-1-pipérazinyl)butyl]pyrrole.

La préparation est effectuée avec une procédure pareille à celle exposée dans l'exemple précédent. On obtient le produit avec un point de fusion 81-84°C.
Les donnés spectroscopiques du composé sont les mêmes déjà exposées aux tableaux 1 et 2.

Exemple 9.- Préparation de 1-[4-(4-Diphénylméthyl-1-pipérazinyl)butyl]pyrazole.

La préparation est effectuée avec une procédure tout à fait semblable à celle exposée dans l'exemple précédent. On obtient ainsi le produit avec un point de fusion 60-64°C.
Son chlorhydrate cristallise dans l'éthanol-éther éthylique, avec un point de fusion 160-165°C.
Les donnés spectroscopiques pour son identification sont exposées dans les Tableaux 1 et 2.

TABLEAU I

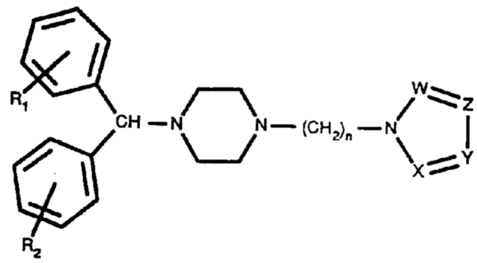

| Exemple nº | $R_1$ | $R_2$ | n | 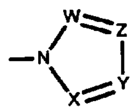 | Méthode | IR ($cm^{-1}$) (KBr) |
|---|---|---|---|---|---|---|
| 1 | H | H | 4 | 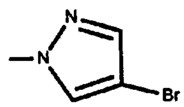 | A | .HCl<br>1456, 950, 919, 762, 706 |
| 2 | H | H | 4 | 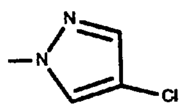 | A | .HCl<br>1487, 1437, 1294, 975, 750, 712 |
| 3 | H | H | 3 | 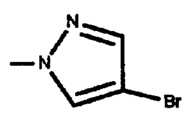 | A | .HCl<br>1656, 1456, 950, 756, 706 |
| 4 | H | H | 4 | 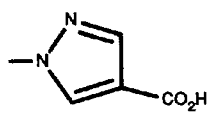 | A<br>C | .HCl<br>1719, 1556, 1430, 1194, 762, 706 |
| 5 | H | H | 4 | 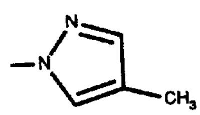 | A | maleate:<br>1694, 1469, 1350, 869, 706, 650 |
| 6 | H | H | 4 | 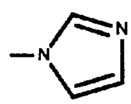 | A | (film)<br>1452, 1283, 1151, 1077, 756, 706, 662 |
| 7 | H | H | 4 | 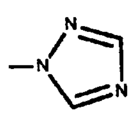 | A | (film)<br>1505, 1492, 1451, 1273, 1139, 1009, 756, 707 |
| 8 | H | H | 4 | 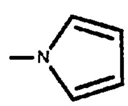 | B<br>C | maleate:<br>1694, 1469, 1350, 869, 744, 706, 650 |
| 9 | H | H | 4 | 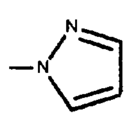 | C | 1449, 1307, 1283, 1140, 1009, 750, 706 |

TABLEAU 2

| Exemple nº | $^1H$-RMN ($CDCl_3$) δ |
|---|---|
| 1 | 1,65 (m,4H); 2,20 (m,10H); 3,8 (t,2H); 4,0 (s,1H); 6,9-7,3 (m,12H) |
| 2 | 1,37 (m,2H); 1,70 (m,2H); 2,33 (m,10H); 3,94 (t,2H); 4,12 (s,1H); 6,9-7,4 (m,12H) |
| 3 | 1,89 (m,2H); 2,17 (t,2H); 2,32 (m,8H); 4,0 (t,2H); 4,12 (s,1H); 7,04-7,37 (m,12H) |
| 4 | 1,37 (m,2H); 1,82 (m,2H); 2,39 (m,10H); 4,08 (t,2H); 4,20 (s,1H); 7,15-7,49 (m,10H); 7,90 (d,2H) |
| 5 | 1,6 (m,4H); 2,05 (s,3H); 2,35 (m,10H); 3,85 (t,2H); 4,1 (s,1H); 6,9-7,3 (m,12H) |
| 6 | 1,46 (m,2H); 1,79 (m,2H); 2,40 (m,10H); 3,91 (t,2H); 4,20 (s,1H); 6,9-7,45 (m,13H) |
| 7 | $d_6$-DMSO: 1,45 (m,2H); 1,80 (m,2H); 2,42 (m,10H); 4,00-4,20 (m,3H); 7,15-7,35 (m,10H); 7,82 (s,1H); 8,25 (s,1H) |
| 8 | 1,50 (m,2H); 1,76 (m,2H); 2,40 (m,10H); 4,09 (t,2H); 4,19 (s,1H); 6,10 (m,2H); 6,61 (m,2H); 7,00-7,40 (m,10H) |
| 9 | 1,46 (m,2H), 1,86 (m,2H); 2,44 (m,10H); 4,11 (t,2H); 4,20 (s,1H); 6,20 (m,1H); 7,17-7,47 (m,12H) |

Activité pharmacologique

Les produits objet de la présente invention sont des puissants antihistaminiques que se caractérisent par le fait d'être exempts d'effets sédatifs, contrairement à la plupart des antihistaminiques connus.

Activité antihistaminique "in vivo"

L'activité antihistaminique a été étudiée en déterminant la protection face à la mortalité induite par le produit 48/80 chez le rat. Cet essai a été réalisé en suivant la technique décrite par C.J.E. Niemegeers et cols. (Arch. int. Pharmacodyn., 234, 164-176 (1978). Les produits objet de la présente invention s'administrent par voie i.p. aux rats. Après 60 minutes on administre le composé 48/80 (0,5 mg/kg, i.v.).

L'activité protectrice se définit comme la survie des rats 4 heures après l'injection i.v. du 48/80.

On étudie l'activité des produits à plusieurs doses à fin de déterminer la dose capable de protéger le 50% des animaux (DE-50).

Ensuite on résume l'activité antihistaminique de quelques uns des produits objet du présent brevet. On compare cette activité avec celle de la difenhidramine, un antihistaminique de référence. La plupart des produits objet de la présente invention sont plus actifs que la difenhidramine, étant donné que leur DE-50 est plus petite.

Activité antihistaminique "in vivo":

Protection de la mort induite par le 48/80

| Exemple nº | DE-50 (mg/kg, i.p.) |
|---|---|
| 1 | 3.2 |
| 2 | 3.0 |
| 3 | 10 |
| 4 | 0.62 |
| 5 | 0.62 |
| 6 | 2.5 |
| 7 | 0.60 |
| 8 | 6.7 |
| Difenhidramine | 5.4 |

Effet sédatif: 1) Test de Irwin

Pour étudier l'abscence d'effet sédatif des produits objet de la présente invention, on les a administré aux rats par voie i.p. et on a observé le comportement des animaux, en suivant les normes décrites dans le test de S Irwin (Science 136, 123-128 (1962)).

On réunit ci-après les résultats obtenus dans les deux évaluations qui reflètent l'effet sédatif:

– Pas.: Passivité, sédation, prostation. Evaluation quantitative entre 0 et 3. On les réalise 1, 2 et 3 heures après le traitement.

– Atax.: Ataxie, on évalue les altérations de coordination dans la locomotion. On les évalue entre 0 et 3. On les réalise 1, 2 et 3 heures après le traitement.

Ci-après on résume les résultats de l'étude de l'effet sédatif de quelques uns des produits objet de la présente invention, à titre d'exemple. Cette activité a été comparée avec celle de la difenhidramine, antihistaminique de référence. Les produits objet de la présente invention ont montré un très faible effet sédatif, contrairement à la difenhidramine qui s'est avérée toxique à la dose de 80 mg/kg, i.p., à cause des effets dépresseurs du SNC.

Effet sédatif: 1) Test de Irwin

| Exemple nº | Dose (mg/kg) | Effet Pas. | Effet Atax. |
|---|---|---|---|
| 1 | (20) | 0.8 | 1.2 |
| | (40) | 0.8 | 1.4 |
| | (80) | Toxique | |
| 2 | (40) | 0.2 | 1.5 |
| | (80) Tox. | 0 | 0.4 |
| 3 | (80) | 0 | 0.7 |
| 4 | (80) | 0.9 | 1 |
| | (160) | 1.2 | 1.6 |
| 5 | (80) | 0.7 | 0.5 |
| 6 | (80) | 1.3 | 0.3 |
| 7 | (80) | Toxique | |
| Difenhidramine | (40) | 0 | 0.9 |
| | (80) | Toxique | |

Effet sédatif: 2) Potentiation du temps de sommeil induit par le pentobarbital

L'étude de la potentiation du temps de sommeil dû au pentobarbital a été réalisée en suivant la méthode décrite par L.E. Allen et cols. (Arz. Forsch. 24, (6), (1974)). Les produits étudiés ont été administrés par voie orale. Une heure plus tard on administre le pentobarbital de sodium (35 mg/kg, s.c.) et on détermine le temps que les animaux tardent à se réveiller. On compare le temps de sommeil avec un groupe d'animaux témoins, traités uniquement avec du pentobarbital de sodium.

A fin de compléter les études qui démontrent l'absence d'effet sédatif des produits objet de la présente invention on a comparé dans ce test l'activité d'un des produits les plus puissants et avec un moindre effet sédatif (exemple 5) avec l'antihistaminique de référence, la difenhidramine. On présente ci-après les résultats de cet essai avec l'exemple 5 et la difenhidramine. Il est évident que la difenhidramine potentialise de façon significative le temps de sommeil à la dose de 20 mg/kg, alors que l'exemple 5 ne potentialise le temps de sommeil induit par le pentobarbital qu'à la dose de 160 mg/kg.

Effet sédatif: 2) Potentialisation du temps de sommeil induit par le pentobarbital

| Exemple nº | Dose (mg/kg, p.o.) | Potentiation temps de sommeil | |
|---|---|---|---|
| 5 | 40 | 15 % | N.S. |
| | 80 | 21 % | N.S. |
| | 160 | 33 % | * |
| Difenhidramine | 10 | 22 % | N.S. |
| | 20 | 38 % | * |

N.S.: Non significatif

* : Différence significative avec le groupe témoin ($p < 0,05$)

On indiquera ci-après, à titre d'exemple, une forme galénique particulière des dérivés objet de la présente invention.

Comprimés

Formule par comprimé

| | |
|---|---|
| Exemple nº 5 | 10,00 mg |
| Lactose | 54,00 mg |
| Amidon de maïs | 26,60 mg |
| Cellulose microcristaline | 18,00 mg |
| Polyvinylpyrrolidone | 6,00 mg |
| Croscarmellose de sodium | 3,60 mg |
| Dioxyde sillicique colloïdal | 0,60 mg |
| Stéarate de magnésium | 1,20 mg |
| | 120,00 mg |

## Revendications

**1.-** Nouveaux dérivés de 1-diphénylméthyl pipérazinyl caracterisés en ce qu'ils répondent à la formule générale I, et leurs sels thérapeutiquement acceptables,

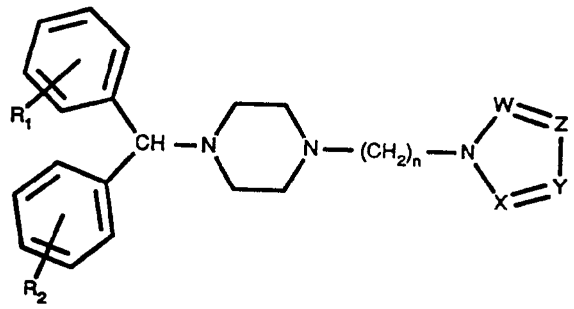

I

dans laquelle:

– $R_1$ et $R_2$, égaux ou différents, représentent un atome d'hydrogène, un halogène, un radical alkyl inférieur, un radical hydroxy, un radical alkoxy, un radical carboxylate d'alkyle, un radical aryl ou aryl substitué,

– n peut avoir les valeurs 2 à 4,

– X, Y, Z et W, égaux ou différents, représentent un atome d'azote ou un atome de carbone lié à un atome d'hydrogène, un halogène ou à un autre radical alkyl, aryl, carboxyalkyl, carboxylique, hydroxyle, alkylhydroxy, sulfonique et alkylsulfonique.

**2.-** Les composés répondant à la formule générale I selon la revendication 1, séléctionnés parmi le groupe suivant:

- 4-Bromo-1-[4-(4-diphénylméthyl pipérazinyl)butyl]pyrazole.
- 4-Chloro-1-[4-(4-diphénylméthyl-1-pipérazinyl)butyl]pyrazole.
- 4-Bromo-1-[3-(4-diphénylméthyl-1-pipérazinyl)propyl]pyrazole.
- 1-[4-(4-Diphénylméthyl-1-pipérazinyl)butyl]1-4-carboxypyrazole.
- 1-[4-(4-Diphénylméthyl-1-pipérazinyl)butyl]-4-méthylpyrazole.
- 1-[4-(4-Diphénylméthyl-1-pipérazinyl)butyl]imidazole.
- 1-[4-(4-Diphénylméthyl-1-pipérazinyl)butyl]-1,2,4-triazole. ,
- 1-[4-(4-Diphénylméthyl-1-pipérazinyl)butyl]pyrrole.
- 1-[4-(4-Diphénylméthyl-1-pipérazinyl)butyl]-4-éthyloxycarbonylpyrazole.
- 1-[4-(4-Diphénylméthyl-1-pipérazinyl)butyl]pyrazole.

**3.-** Procédé de préparation des composés selon l'une des revendications 1 et 2, caracterisé par la mise en oeuvre d'au moins l'une des opérations suivantes:

3a.-Par réaction d'un composé de formule générale IIa

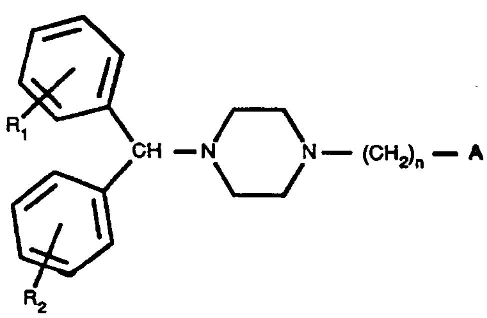

IIa

ou bien IIb

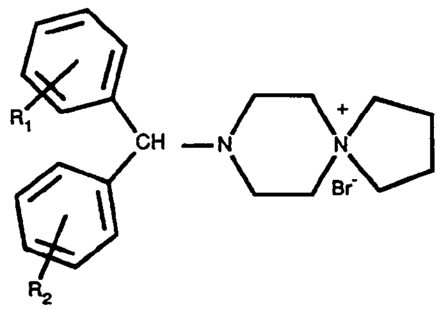

IIb

dans lesquelles $R_1$ , $R_2$ et n ont les significations mentionnées précédemment, et A représente un atome d'halogène, ou un bon "groupe partant" choisi parmi le tosyloxy ou le mésyloxy, avec un composé de formule générale III

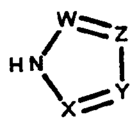

III

dans laquelle X, Y, Z et W ont les significations mentionnées précédemment;
3b.- Par réaction d'un composé de formule générale IIa, dans laquelle A représente un radical $-NH_2$, avec le 2,5--diméthoxytétrahydrofurane;
3c.- Par réaction d'un composé de formule générale IV

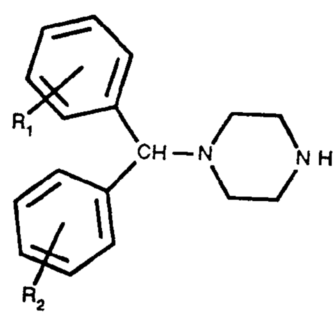

IV

dans laquelle $R_1$ et $R_2$ ont les significations mentionnées précédemment, avec un composé de formule générale V

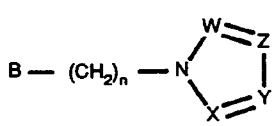

V

où X, Y, Z, W et n ont les significations mentionnées précédemment, et B représente un atome d'halogène, ou un bon "groupe partant" choisi parmi le tosyloxy ou le mésyloxy;

**4.-** A titre de médicaments, les derivés de formule générale I et leurs sels thérapeutiquement acceptables, selon les revendications 1 ou 2 en particulier à titre de médicaments utilisés comme antihistaminiques.

**5.-** Compositions pharmaceutiques, caracterisées par le fait qu'elles contiennent, outre un support pharmaceutiquement acceptable, au moins un dérivé de formule générale I ou l'un de ses sels physiologiquement acceptables, selon l'une des revendications 1 et 2

**6.-** Utilisation des dérivés de formule générale I et ses sels physiologiquement acceptables, selon l'une des revendications 1 et 2, pour la fabrication de médicaments destinés à la prophilaxie et au traitement de diverses maladies allergiques provoquées par l'histamine.

Office européen des brevets

# RAPPORT DE RECHERCHE EUROPEENNE

Numero de la demande

EP 91 40 2056

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | CHEMICAL ABSTRACTS, vol. 108, no. 15, 11 avril 1988, page 763, résumé no. 131862d, Columbus, Ohio, US; & ES-A-541 562 (ALCALOIDES ABELLO S.A.) 01-04-1986 * Extrait * | 1,3-6 | C 07 D 231/16<br>C 07 D 231/14<br>C 07 D 207/32<br>C 07 D 403/06<br>A 61 K 31/41<br>A 61 K 31/40 |
| X | CHEMICAL ABSTRACTS, vol. 107, no. 1, 6 juillet 1987, page 668, résumé no. 7214j, Columbus, Ohio, US; & ES-A-541 563 (ALCALOIDES ABELLO S.A.) 16-12-1985 * Extrait * | 1,3-6 | |
| X | CHEMICAL ABSTRACTS, vol. 106, no. 3, 19 janvier 1987, page 614, résumé no. 18620u, Columbus, Ohio, US; & ES-A-541 561 (ALCALOIDES ABELLO S.A.) 16-12-1985 * Extrait * | 1,3-6 | |
| A | CHEMICAL ABSTRACTS, vol. 113, no. 17, 22 octobre 1990, page 767, résumé no. 152278m, Columbus, Ohio, US; & JP-A-02 124 871 (DAINIPPON PHARMACEUTICAL CO., LTD) 14-05-1990 * Extrait * | 1,3,4 | **DOMAINES TECHNIQUES RECHERCHES (Int. Cl.5)** |
| A | ARZNEIMITTEL FORSCHUNG, vol. 37, no. 10, octobre 1987, pages 1103-1107; S. GUBERT et al.: "Synthesis of some N-benzhydrylpiperazine derivatives as calcium antagonists" * Article en entier * | 1,3,4 | C 07 D 231/00<br>C 07 D 207/00<br>C 07 D 233/00<br>C 07 D 249/00<br>C 07 D 295/00<br>C 07 D 403/00<br>A 61 K 31/00 |
| D,A | EP-A-0 058 146 (U.C.B. S.A.) * Revendications * | 1,3,5 | |

Le présent rapport a été établi pour toutes les revendications

| Lieu de la recherche | Date d'achèvement de la recherche | Examinateur |
|---|---|---|
| LA HAYE | 22-10-1991 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)